# EUROPEAN PATENT APPLICATION

(11) **EP 4 792 753 A1**
(43) Date of publication of application: **19.08.2026**
(21) Application number: 26158808.1
(22) Date of filing: 16.02.2026
(51) Int. Cl.: A61B 17/062

(54) **SURGICAL SUTURING DEVICE**

(30) Priority: 17.02.2025 NL 2039783
(71) Applicant: Mellon Medical B.V., 2628XJ Delft (NL)
(72) Inventor: VAN EKEREN, Roel, 2628 XJ DELFT (NL); MULDER, Julian, 2628 XJ DELFT (NL); ZANDMAN, Jonathan Johannes, 2628 XJ DELFT (NL)
(74) Representative: Arnold & Siedsma

(57) **Abstract**

A surgical suturing device for passing a double-ended surgical needle back and forth is disclosed herein. The device comprises a pair of jaw members movable relative to each other between an open state and a closed state. Each of the jaw members is provided with a needle-locking element that is movable between a locking position and a release position for alternately locking and releasing a respective end of the needle. The device further comprises an alternator system arranged to move the needle-locking elements between their locking and release positions for locking one end of the needle and releasing the other end of the needle. The alternator system comprises an alternator element that is movable from a first position to a second position for moving one of the needle-locking elements to its release position, and from the second position to the first position for moving the other needle-locking element to its release position.

## Description

The present invention relates to a surgical suturing device for passing a double-ended surgical needle back and forth.

Suturing is a technique in surgery that involves the precise placement of sutures to close wounds or surgical incisions. In surgical procedures like laparotomy, optimal results can be achieved with the use of suturing devices.

Such a surgical suturing device is known from, e.g., European patent No. 3 426 165, which discloses a surgical suture apparatus for passing a double-ended surgical needle forward and backwards. The surgical apparatus comprises a first jaw element and a second jaw element, and an operating device to operate a first holding device in the first jaw element and a second holding device in the second jaw element to alternately hold a first needle-end by the first holding device and a second needle-end by the second holding device. The first jaw element and second jaw element are movable with respect to each other between a closed position and an open position for arranging tissue to be sutured between the jaw elements as described further below. The operating device comprises a switch device tiltable between a first operating position, in which a needle is held by the first holding device, and a second operating position, in which the needle is held by the second holding device, and an activation device configured to tilt the switch device between the first operating position and the second operating position.

A needle-holding system for alternately locking and releasing an end of a double-ended surgical needle is known from, e.g., European patent No. 3 784 145, which discloses a needle holder to hold a needle-suture combination that comprises a double-ended needle and a suture connected to the needle between the two needle ends, wherein each of the needle ends comprises a locking groove. The needle holder comprises a needle channel arranged to receive a respective needle end, the needle channel comprising an inlet to introduce the respective needle end into the needle channel, and a needle locking element to be received in the locking groove of the needle end to lock the respective needle end in the needle channel.

In the open position the first jaw element and the second jaw element and therewith the first holding device and second holding device are spaced further from each other. The distance between the first holding device and the second holding device is substantially larger than the length of the surgical needle so that the surgical apparatus can be manipulated to arrange tissue to be sutured between the needle end that is not held by one of the holding devices, and the other holding device. Then, by further manipulation of the apparatus, the needle can be moved through the tissue at a suitable location at least until the free end of the needle comes out of the tissue. Then, the apparatus can be actuated by an actuation force to move the first and second jaw element to the closed position and to pass the needle from one of the holding devices to the other holding device so that a suture is provided through the tissue.

The activation device comprises a hammer movable in a longitudinal direction of the apparatus. The hammer comprises a pusher element configured to cooperate with a first cam profile and a second cam profile on the switch device. The first cam profile is shaped to move the switch device from the first operating position to the second operating position when the pusher element is pushed into this first cam profile. Correspondingly, the second cam profile is shaped to move the switch device from the second operating position to the first operating position when the pusher element is pushed into this second cam profile. To ensure that the switch device remains either in the first operating position or in the second operating position as long as the activation device is not activated to switch the switch device between the first operating position and the second operating position, permanent magnets are provided at fixed positions and configured to cooperate with magnetic parts of the switch device to hold the switch device in the first operating position or second operating position, respectively.

While the devices disclosed in EP 3 426 165 and EP 3 784 145 include considerable improvements with respect to prior suturing devices, they also have certain limitations.

It is an object of the present invention, amongst other objects, to provide a further improved surgical suturing device wherein the limitations of known devices are at least partially overcome.

Thereto, according to an aspect of the present disclosure, a surgical suturing device for passing a double-ended surgical needle back and forth is provided, wherein the device comprises:
- a pair of jaw members movable relative to each other between an open state and a closed state, wherein each of the jaw members is provided with a needle-locking element that is movable between a locking position and a release position for alternately locking and releasing a respective end of the needle;
- an alternator system arranged to move the needle-locking elements between their locking and release positions for locking one end of the needle and releasing the other end of the needle, wherein the alternator system comprises an alternator element that is movable from a first position to a second position for moving one of the needle-locking elements to its release position, and from the second position to the first position for moving the other needle-locking element to its release position; and
- an alternator spring arranged to bias the alternator element to alternately the first position and the second position.

As the alternator system moves the needle-locking elements between their locking and release positions in the closed position of the pair of jaw members, a needle that is locked at one end by the needle-locking element of one of the jaw members can be passed to the other jaw member so that a suture is provided through the tissue. Preferably, in the first position of the alternator element, one of the needle-locking elements is in its locking position and the other needle-locking element is in its release position while, in the second position, the one needle-locking element is in its release position and the other needle-locking element is in its locking position.

By arranging a spring to bias the alternator element to its respective positions, it can be ensured that the alternator element is biased to either position throughout most of its movement between its positions. This way, the so-called 'dead zone' can be minimised. The dead zone is the region where the force intended to keep the alternator element in either position is insufficient to reliably bias the alternator element to either position. By arranging a spring to bias the alternator element to its respective positions, the dead zone can be at least reduced when compared to the aforesaid prior art in which magnets are used, whose attraction force quickly declines with the distance thereto due to the inverse relation between the magnetic field strength and the distance to the magnet. As the dead zone is minimised, the alternator element can be easily moved therethrough from the first position to the second position and vice versa, and bi-stability of the alternator system can be ensured. In particular, it can be prevented that the needle is accidentally released from the device or that both needle ends remain locked at the same time such that the device is fastened to the tissue. This enables a more accurate and/or reliable needle-handling during the suturing operation, thereby improving the overall performance of the suturing device. Moreover, the alternator system can comprise few parts, be easily assembled, and be made without magnets or other magnetic components, which is particularly convenient in a surgical setting.

According to a preferred embodiment of the surgical suturing device, the alternator spring is preloaded. This way, the bi-stability can be ensured.

According to a further preferred embodiment of the surgical suturing device, the alternator spring is a leaf-spring. As such, the alternator spring can guide movement of the alternator element between its positions and constrain motions of the alternation element that deviate from the trajectory from the first position to the second position and vice versa. In addition, the alternator spring being a leaf-spring enables a compact configuration of the alternator system. Furthermore, particularly if the alternator leaf-spring is preloaded, it can be ensured that the alternator spring exerts a relatively constant force on the alternator element throughout its movement between its respective positions. This way, both the dead zone and the operating force required to move the alternator element between its positions can be minimised.

Additionally, or alternatively, a compact configuration can be provided by arranging the alternator spring to push against the alternator element.

A compact configuration can further be provided if the alternator element is tiltable about a tilt axis between the first position and the second position. Preferably, the alternator spring is arranged to apply a torque to the alternator element about the tilt axis in a first torque direction in the first position and, in the second position, in a second torque direction opposite to the first torque direction. Likewise, it is preferred if the tilt axis is perpendicular to a plane spanned by the pair of jaw members and/or is perpendicular to the angle bisector of the jaw members as seen in said plane spanned by the jaw members.

A further preferred embodiment of the surgical suturing device comprises at least one end stop surface, wherein the alternator element is arranged to rest against the least one end stop surface in the first position and/or the second position. This way, stability of the alternator element in the first position and/or the second position is enhanced. Preferably, the surgical suturing device comprises a first and a second end stop surface, wherein the alternator element is arranged to rest against the first end stop surface in the first position and against the second end stop surface in the second position.

A further preferred embodiment of the surgical suturing device comprises a trigger system arranged to move the alternator element between the first position and the second position. Preferably, the trigger system comprises a trigger element arranged to engage the alternator element to move the alternator element between its positions. Thereto, the alternator element may comprise a first cam profile and a second cam profile, wherein the trigger element is arranged to alternately engage the first cam profile to move the alternator element from the first position to the second position, and the second cam profile to move the alternator element from the second position to the first position.

The use of profiles on the alternator element, wherein the trigger element is arranged to alternately follow the shape of the respective profiles to alternate the alternator element between its positions, provides a reliable operation of the alternator system. Various shapes for the profiles can be envisaged. Alternatively, the trigger element may comprise first and second profiles, wherein the alternator element is arranged to alternately engage the first profile and the second profile to move the alternator element between its positions.

The trigger system may comprise an operating element manually movable between a neutral position and a triggered position for operating the trigger system to move the alternator element between the first position and the second position. The operating element is preferably biased to the neutral position and is preferably arranged to be actuated by pressing it. The trigger element may be arranged to be moved into engagement with either cam profile upon manually moving the operating element. For example, the operating element may be a button that is movable relative to the jaw members.

According to a further preferred embodiment of the surgical suturing device, the trigger element is biased to a neutral position in which the trigger element is positioned to engage the first cam profile in the first position of the alternator element and to engage the second cam profile in the second position of the alternator element.

By biasing the trigger element to a neutral position in which the trigger element is positioned to engage the first cam profile in the first position of the alternator element and to engage the second cam profile in the second position of the alternator element, it can be ensured that the trigger element is properly positioned to move the alternator element between its position when actuating the trigger system. The trigger system may comprise a trigger spring arranged to bias the trigger element to the neutral position.

A trigger system as described above can also be implemented in a surgical suturing device without the alternator spring. As such, according to a further aspect of the present disclosure, a surgical suturing device for passing a double-ended surgical needle back and forth is provided, preferably as described above, wherein the device comprises:
- a pair of jaw members movable relative to each other between an open state and a closed state, wherein each of the jaw members is provided with a needle-locking element that is movable between a locking position and a release position for alternately locking and releasing a respective end of the needle;
- an alternator system arranged to move the needle-locking elements between their locking and release positions for locking one end of the needle and releasing the other end of the needle, wherein the alternator system comprises an alternator element that is movable between a first position, in which one of the needle-locking elements is in its locking position and the other needle-locking element is in its release position, and a second position in which the one needle-locking element is in its release position and the other needle-locking element is in its locking position, wherein the alternator element comprises a first cam profile and a second cam profile;
- a trigger system arranged to move the alternator element between the first position and the second position, wherein the trigger system comprises a trigger element arranged to alternately engage the first cam profile to move the alternator element from the first position to the second position, and the second cam profile to move the alternator element from the second position to the first position, wherein the trigger element is biased to a neutral position in which the trigger element is positioned to engage the first cam profile in the first position of the alternator element and to engage the second cam profile in the second position of the alternator element.

According to a preferred embodiment of the surgical suturing device, the trigger element comprises at least one flat engaging portion, preferably a first flat engaging portion and a second flat engaging portion, wherein the trigger spring is arranged to press the at least one flat engaging portion of the trigger element against a further flat engaging portion to bias the trigger element to the neutral position. As such, the trigger element can be efficiently biased to its neutral position.

Preferably, the trigger spring is a leaf-spring comprising a trigger-engaging surface arranged to press against the flat engaging portion of the trigger element to bias the trigger element to the neutral position. The trigger spring being a leaf-spring enables a compact configuration of the trigger system and ensures that the trigger element can be biased in a stable manner. It is further preferred if the trigger-engaging surface is arranged to press against the first flat engaging portion of the trigger element to press the second flat engaging portion of the trigger element against said further flat engaging portion of the surgical suturing device. In a preferred embodiment, the trigger spring may be biased by a second leaf-spring to ensure that the trigger-engaging surface of the trigger leaf-spring is flat when in the neutral position to keep the trigger element biased to its neutral position.

Said operating element may be a button or the like. A preferred embodiment of the surgical suturing device comprises an opening spring arranged to bias the pair of jaw members to the open state with a first force, and an operating spring arranged to bias the operating element to the neutral position with a second force, wherein the first force is smaller than the second force. This way, a reliable and relatively simple operating device of the surgical device is obtained which uses a single actuation of the operating element to move the pair of jaw members into the closed state and to subsequently operate the actuator system to pass the surgical needle from one of the jaw members to the other. As such, the surgical suturing device can be easily held and operated by a single hand of a user, leaving the other hand free for other tasks.

It is preferred if the operating element is arranged on one of the jaw members, preferably at a side facing away from the other jaw member. By arranging the operating element on this side, the user can directly manipulate the respective jaw member and thus obtain direct feedback of the position of the jaw member and the force being exerted thereon. Also, the user has a large flexibility and feeling in manipulating the device in different positions with respect to a patient, since the position and orientation of the hand of the user is directly linked with the position and orientation of the jaw member.

The operating spring may be pretensioned between the operating element and the one jaw member on which the operating element is arranged. The opening spring may be pretensioned between the operating element and said other jaw member.

Any of the aforementioned springs may be any element that is capable of converting kinetic energy into potential energy and vice versa. The spring may for example comprise a helical spring or a leaf spring, or an elastic element of rubber or a rubber-like material.

The surgical suturing device is preferably a hand-held surgical suturing device. It is further preferred if the pair of jaw members has a tweezer-like configuration, such that pinching actuation forces on the jaw members at mutually opposite sides of the device result in a movement of the jaw members towards each other into the closed state, and subsequently in operating the alternator system by actuation of the operating element. The pinching actuation force on the operating element is preferably provided by the thumb and/or thenar while the other jaw member is held by one or more of the other fingers of the same hand. By holding the device in this way, the device can be properly manipulated by the user, and the user has a good view of the suturing operation. For example, the user may be able to hold the surgical device within the hand palm and provide the actuation force via the thumb on one side and the index and/or middle finger on the other side.

A preferred embodiment of the surgical suturing device further comprises a toggle element arranged for manually moving the alternator element between the first position and the second position in the open state of the pair of jaw members. The toggle element may be separate from the trigger system and operable independently of the trigger system. When moving the alternator element in the open state using such a toggle element, the needle can be released on one end without also being locked at its other end. As such, the toggle element allows the user to conveniently release the needle from the surgical suturing device after a suturing operation.

A toggle element as described above can also be implemented in a surgical suturing device without the alternator spring or even without an alternator system as described above. As such, according to a further aspect of the present disclosure, a surgical suturing device for passing a double-ended surgical needle back and forth is provided, preferably as described above, wherein the device comprises:
- a pair of jaw members movable relative to each other between an open state and a closed state, wherein each of the jaw members is provided with a needle-locking element that is movable between a locking position and a release position for alternately locking and releasing a respective end of the needle;
- a toggle element arranged for manually moving at least one of the needle-locking elements from its locking position to its release position in the open state.

Preferably, the toggle element comprises a manually operable end, positioned between the jaw members for manually operating the toggle element in the open state. As the operable end of the toggle element is positioned between the jaw members, its accessibility is reduced in the closed state of the pair of jaw members, such that the risk of accidentally operating the toggle element can be reduced. It is then further preferred if the operable end extends exclusively within an outer contour of the pair of jaw member in the closed state. This way, the operable end may be only accessible in the open state of the pair of jaw members and the risk of accidentally operating the toggle element can be further minimised.

According to a further preferred embodiment of the surgical suturing device, at least one, preferably each, of the needle-locking elements is biased to its locking position, or the alternator system is arranged to alternately push directly against either needle-locking element during movement of the alternator element between the first position and the second position to move the respective needle-locking element to its locking or release position.

According to a further aspect of the present disclosure, a surgical suturing device for passing a double-ended surgical needle back and forth is provided, preferably as described above, wherein the device comprises:
- a pair of jaw members movable relative to each other between an open state and a closed state, wherein each of the jaw members is provided with a needle-locking element that is movable between a locking position and a release position for alternately locking and releasing a respective end of the needle;
- an alternator system arranged to move the needle-locking elements between their locking and release positions for locking one end of the needle and releasing the other end of the needle, wherein at least one of the needle-locking elements is biased to its locking position and the alternator system is arranged to push directly against the at least one needle-locking element to move the respective needle-locking element to its release position.

This aspect is based on the novel insight that the feature that the needle-locking element is biased to its locking position, in combination with the feature that the alternator system pushes against the needle-locking element to move the respective needle-locking element to its release position, enhances accuracy and/or reliability of the needle-handling to an unforeseen extent.

Preferably, each of the needle-locking elements is biased to its locking position, wherein the alternator system is arranged to push directly against either needle-locking element, preferably during movement of the alternator element between the first position and the second position, to move the respective needle-locking element to its release position.

According to a further preferred embodiment of the surgical suturing device, the alternator system further comprises a rocker element arranged between the alternator element and one of the needle-locking elements to push against the respective needle-locking element. Due to a possible asymmetry of the surgical suturing device and in particular the alternator system, the distance between the alternator element and one or both of the locking elements may differ in respectively the closed state and the open state. The rocker element can compensate for such a distance difference.

The rocker element arranged between the alternator element and the respective needle-locking element thus allows the pair of jaw members to move to the open state. This is particularly advantageous in a configuration wherein the alternator system is arranged to push directly against either needle-locking element during movement of the alternator element between the first position and the second position to move the respective needle-locking element to its release position, in particular as described above.

A rocker element as described above can also be implemented in a surgical suturing device without the alternator spring. As such, according to a further aspect of the present disclosure, a surgical suturing device for passing a double-ended surgical needle back and forth is provided, preferably as described above, wherein the device comprises:
- a pair of jaw members movable relative to each other between an open state and a closed state, wherein each of the jaw members is provided with a needle-locking element that is movable between a locking position and a release position for alternately locking and releasing a respective end of the needle;
- an alternator system arranged to move the needle-locking elements between their locking and release positions for locking one end of the needle and releasing the other end of the needle, wherein the alternator system comprises an alternator element that is movable between a first position, in which one of the needle-locking elements is in its locking position and the other needle-locking element is in its release position, and a second position in which the one needle-locking element is in its release position and the other needle-locking element is in its locking position, wherein the alternator system further comprises a rocker element arranged between the alternator element and one of the needle-locking elements to push against the respective needle-locking element.

Preferably, the alternator element engages the rocker element at an alternator engagement point which, in the open state, is spaced from a longitudinal axis of the jaw member that is provided with the respective needle-locking element. It is then further preferred if the rocker element extends from the alternator engagement point to the longitudinal axis of the jaw member.

According to yet another aspect of the present disclosure, a needle-holding system for alternately locking and releasing an end of a double-ended surgical needle is provided, wherein the system comprises:
- a needle channel arranged to accommodate the needle end, wherein the needle channel comprises a needle inlet arranged for inserting the needle end into the needle channel;
- a needle-locking element arranged movable between a locking position and a release position for alternately locking and releasing the needle end in the needle channel, wherein the needle-locking element is provided with a needle-locking opening arranged for receiving the needle end in the release position and accommodating the needle end in the locking position, wherein the needle-locking element is further arranged to, upon inserting a needle end into the needle-locking opening in the locking position, move from a locking state to a loading state in which the needle-locking element is configured to receive the needle end in the needle-locking opening.

The needle-holding system may be provided in a surgical suturing device for passing a double-ended surgical needle back and forth, as described above.

By arranging the needle-locking element to, upon inserting a needle end into the needle-locking opening in the locking position, move from the locking state to a loading state, a needle end can be loaded into the needle-holding system even if the locking element is in its locking position. This way, the needle can be conveniently loaded into the needle-holding system at the start of a suturing operation without the need to first check whether the needle-locking element of the needle-holding system is in the release position and to, if not, move the needle-locking element to the release position before inserting the needle end into the needle-locking opening. Hence, the needle may be conveniently inserted into one of the needle-holding systems regardless of the position of its locking element, after which said jaws can be closed such that the needle is automatically loaded and locked in the needle-holder whose locking element was already in the locking position. As such, an improved surgical suturing device can be provided.

The needle-locking element may be arranged to, upon inserting a needle end into the needle-locking opening in the locking position, move from the locking position to the release position.

According to a preferred embodiment of the needle-holding system, the needle-locking element is elastically deformable in the locking position from the locking state to the loading state upon inserting a needle end into the needle-locking opening. As the needle-locking element is elastically deformable, the needle-locking element can automatically return to its locking state to lock the needle end after insertion. This facilitates loading the needle into the needle-holding system in such a way that the needle and the needle-holding system are directly ready for use in a suturing operation. More in particular, the needle-locking element may be deformable such that the locking opening is expanded in the loading state and thereby sized to receive the needle end.

As the end of a surgical needle typically tapers, the needle-locking element may be forced radially outwards as the tapered needle end is inserted into the needle-locking opening.

According to a further preferred embodiment of the needle-holding system, the needle-locking opening has a release section, aligned with the needle channel in the release position, and a locking section, aligned with the needle channel in the locking position. The release section is sized for receiving the needle end. In particular, the release section may be wider than the locking section.

Preferably, in the loading state, the locking section is wider than in the locking state and the needle-locking element is configured to receive the needle end in the locking section.

According to a further preferred embodiment of the needle-holding system, the elongate needle-locking element comprises a pair of prong parts defining the needle-locking opening between the prong parts. As the needle-locking opening is defined between two prong parts, the needle-locking element can be deformed to the loading state in a relatively easy manner, as the prong parts may be outwardly bendable for expanding the needle-locking opening therebetween.

Preferably, the locking section of the needle-locking opening is located between the release section and a free end of the pair of prong parts. The prong parts are bendable more easily by applying a force at the free ends of the prong parts than by applying a force closer to their base ends connected to the rest of the needle-locking element. As the locking section, in comparison with the release section, is closer to the free ends of the prong parts, the needle-locking element can be conveniently moved to its loading state by inserting a needle end into the locking section with relatively little force.

Preferably, the locking section of the needle-locking opening is narrower than the diameter of the needle at the location where the needle end is to be locked. This way, play can be minimised to ensure a reliable locking of the needle end.

The needle-locking element may be elongate, wherein an end of the elongate needle-locking element is provided with the needle-locking opening.

Further, at least a portion of the needle-locking element may be plate-like, wherein the plate-like portion is provided with the needle-locking opening and is perpendicular to the needle channel. Preferably, the needle-locking element has a plate-like end provided with the needle-locking opening.

The needle-locking element is preferably movable between the locking position and the release position along a first direction, which is preferably transverse to the needle channel. For example, the needle-locking element may be slidable between the locking position and the release position along a direction that is radial with respect to the needle channel. It is further preferred if the needle-locking element is movable between the locking state and the loading state along a second direction. For example, the needle-locking element may be outwardly expandable in the second direction. The second direction is preferably transverse to both the first direction and the needle channel. The plate-like end of the locking element may thus be longitudinally slidable between its two positions and laterally movable between its two states.

The needle-holding system may comprise a locking element channel in which the needle-locking element is slidably arranged. This way, movement of the needle-locking element between its positions can be guided in a secure manner. Preferably, the locking element channel is perpendicular to the needle channel. More in particular, the orientation of the locking element channel preferably corresponds to said first direction.

During a suturing operation, not only the needle end but also tissue may enter the needle channel as the needle end moves into and out of the needle channel. Therefore, according to a further preferred embodiment of the needle-holding system, the locking element channel is open at an end for discharging tissue. Additionally, or alternatively, the needle channel may further comprise a needle channel outlet for discharging tissue from the needle channel. Preferably, the needle channel outlet and the needle inlet are arranged at mutually opposite ends of the needle channel.

Further provided is a surgical suturing device for passing a double-ended surgical needle back and forth, preferably as described above, wherein the device comprises a pair of jaw members movable relative to each other between an open state and a closed state, wherein each of the jaw members is provided at its free end with a needle-holding system arranged for alternately locking and releasing a respective end of the needle, wherein at least one, preferably each, of the needle-holding systems is a needle-holding system as described above.

Preferably, the needle-locking element is preferably elongate and may extend in the longitudinal direction of the respective jaw member.

The needle-locking element may be movable towards the free end of the respective jaw member from the locking position into the release position, and movable away from the free end of the respective jaw member from the release position into the locking position. Preferably, the needle-locking element is biased to the locking position.

Further provided is a method of loading an end of a surgical needle into a needle-holding system prior to a suturing operation, wherein the method comprises the step of inserting the needle end into the needle-locking opening of the needle-locking element of the needle-holding system while the needle-locking element is in its locking position. Preferably, the needle-holding system is a needle-holding system as described above.

Preferably, the locking section of the needle-locking opening is narrower than the diameter of the needle at the location of its locking groove. This way, play can be minimised to ensure a reliable locking of the needle end.

It is to be appreciated that various combinations of the features of the various aspects described herein can be envisaged, as illustrated in the following, wherein the various aspects of the surgical suturing device are further illustrated with reference to the appended drawings, wherein:
- Figures 1A-D represent a stepwise illustration of the transfer of a double-ended surgical needle from one of the jaw members of the surgical suturing device to the other;
- Figure 2 represents a sectional view of the surgical suturing device;
- Figures 3A-C represent a stepwise illustration of the alternating of the needle-locking elements of the surgical suturing device between their locking and release positions;
- Figures 4A-D represent a stepwise illustration of the biasing of the trigger system of the surgical suturing device to a neutral position;
- Figures 5A-B illustrate the torque exerted by the alternator spring on the alternator element in the respective positions of the alternator element;
- Figures 6A-D illustrate the alternator system of the surgical suturing device in its respective positions in respectively the closed state and the open state of the jaw members;
- Figures 7A-C represent a stepwise illustration of the locking of a needle end by a needle-holding system of the surgical suturing device during normal operation;
- Figures 8A-C represent a stepwise illustration of the loading of a needle end into the needle-locking element of the needle-holding system at the start of a suturing operation.

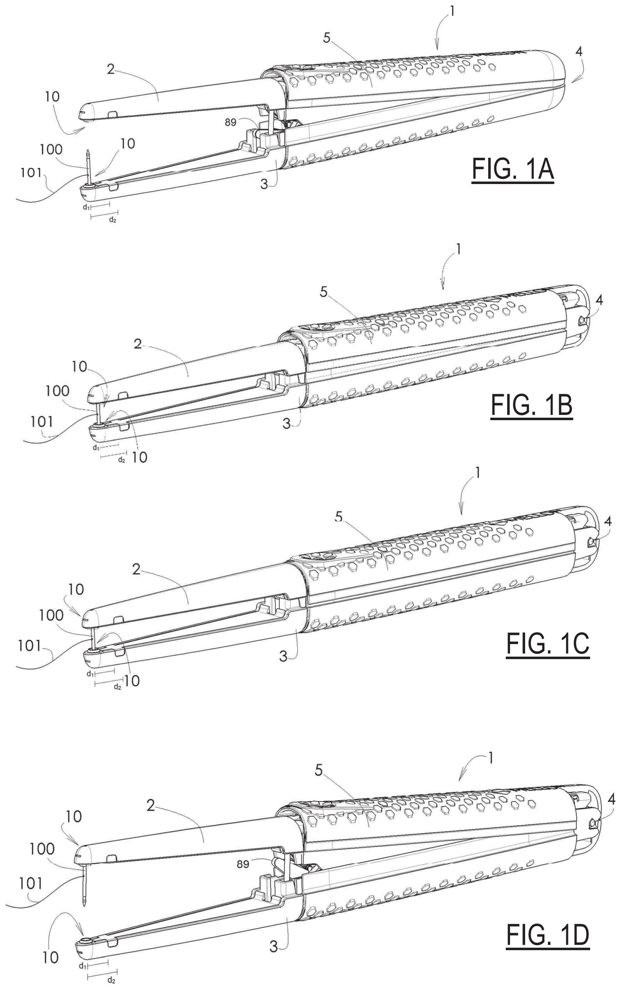

In Figures 1A-D, a hand-held surgical suturing device 1 is shown. The device 1 comprises an upper jaw member 2 and a lower jaw member 3, which are pivotable relative to each other about a hinge connection 4 from an open state, as shown in Figure 1A, to a closed state as shown in Figure 1B. Each jaw member 2, 3 is provided at its free end with a needle holder 10. In Figures 1A-B, a double-ended surgical needle 100, with a suture 101 attached thereto, is held by the needle holder 10 in the lower jaw member 3.

On the upper jaw member 2, a button 5 is provided that is pivotable relative to the jaw members 2, 3 about the hinge connection 4 between a neutral position, as shown in Figures 1A-B, and a triggered position, as shown in Figure 1C. When pushing the button 5 in the closed state, the needle 100 is released by the needle holder 10 holding it and is locked by the needle holder 10 in the other jaw member 2, such that the needle 100 is transferred between the jaw members 2, 3. The jaw members 2, 3 then return to the open state as shown in Figure 1D, wherein the needle 100 is now held by the needle holder 10 in the upper jaw member 2.

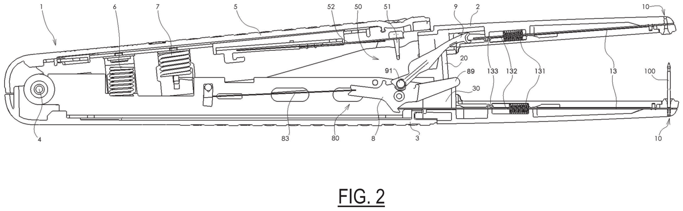

Figure 2 represents a sectional view of the surgical suturing device 1. The device 1 comprises an opening spring 6 pretensioned between the button 5 and the lower jaw member 3 to bias the jaw members 2, 3 to the open state, and an operating spring 7 pretensioned between the button 5 and the upper jaw member 2 to bias the button 5 to its neutral position with a second force. The force biasing the jaw members 2, 3 to the open state is smaller than the force biasing the button 5 to its neutral position. This way, when pressing the button 5 in the open state, the jaw members 2, 3 move to the closed state before the button 5 is triggered. Movement of the jaw members 2, 3 between the open state and the closed state is guided by a pair of guiding portions 20, 30 respectively connected to the jaw members 2, 3.

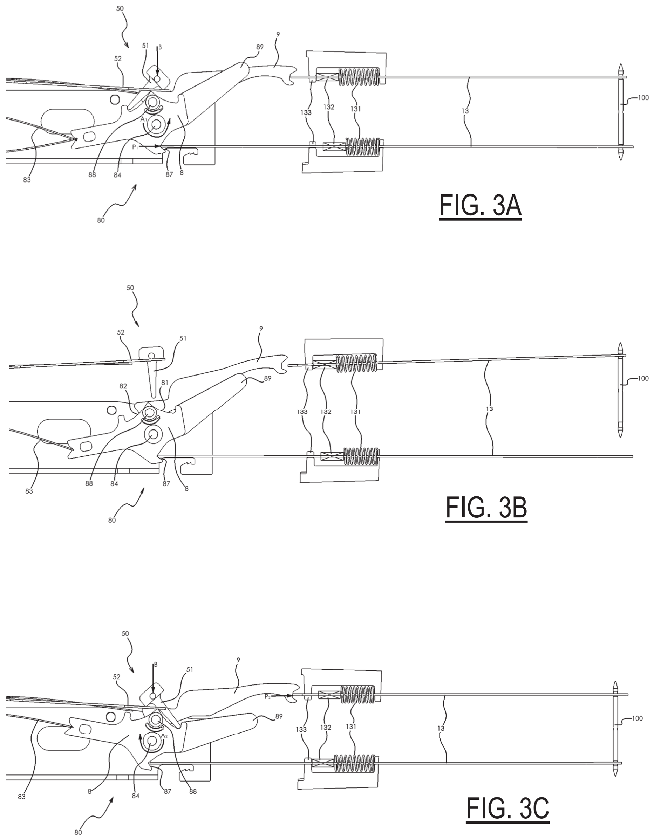

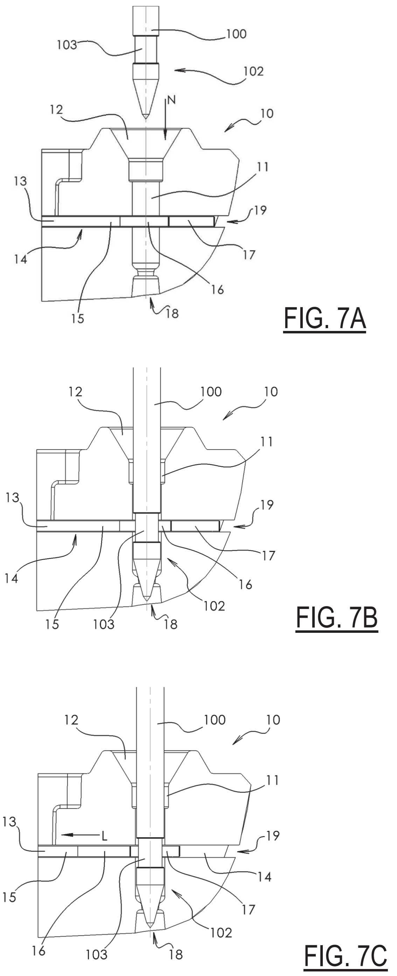

The surgical suturing device 1 further comprises an alternator system 80 for actuating the locking and releasing of a needle by the needle holders 10. In the following, the alternator system 80 is described in more detail with reference to Figures 3A-6B. The surgical suturing device 1 comprises a trigger system 50 for manually operating the alternator system 80, as described in more detail with reference to Figures 3A-4D. In Figures 7A-8C, one of the needle holders 10 is illustrated in more detail.

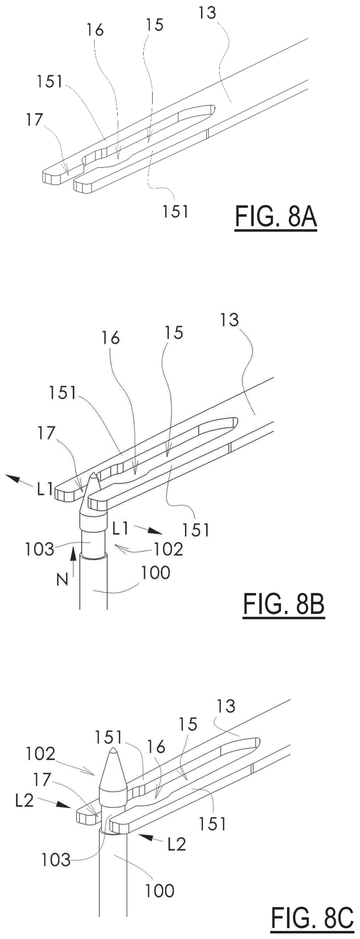

Figures 7A-C represent a stepwise illustration of the locking of an end 102 of the needle 100 in the needle holder 10. The holder 10 comprises a needle channel 11 with an inlet 12, and an elongate needle-locking element 13 slidably arranged in a locking element channel 14. As shown in Figure 8A, the end of the elongate locking element 13 is plate-like and is provided with a needle-locking opening 15 for receiving and accommodating the needle end 102. The locking opening 15 has a wide section 16 and a narrow section 17. The remainder of the locking element 13 is rod-shaped.

In Figure 7A, the locking element 13 is in a release position, in which the wide section 16 is aligned with the needle channel 11 for receiving the needle end 102 through the inlet 12, as indicated by arrow N, into the needle channel 11 and the locking opening 15. In Figure 7B, the needle end 102 is received in the locking opening 15. Hereto, the wide section 16 is wider than the needle end 102 and the needle-holding portion of the needle channel 11. The locking element 13 is then slid into a locking position as indicated by arrow L in Figure 7C. In the locking position, the narrow section 17 of the locking opening 15 is aligned with the needle channel 11. In the locking position, the locking element 13 is received in a circumferential groove 103 on the needle end 102 and thereby locks the needle end 102 in the needle channel 11. Hereto, the narrow section 17 is narrower than the outer diameter of the needle end 102 and preferably also narrower than the diameter of the needle end 102 at the location of the groove 103. To release the needle end 102, the locking element 13 is slid back to the release position shown in Figure 7B such that the needle end 102 can be removed from the needle channel 11 upon moving the jaw members 2, 3 to the open state.

During a suturing operation, not only the needle end 102 but also tissue may enter the needle channel 11 via the inlet 12, as the needle end 102 moves into and out of the needle channel 11. To prevent the tissue from accumulating in the needle channel 11 and thereby the needle end 102 from jamming in the needle channel 11, each of the channels 11, 14 has an open end 18, 19 for discharging tissue.

Furthermore, as shown in Figures 1A-D, each of the jaw members 2, 3 is provided at its free end with a mark 21, 31 indicating respective distances d₁, d₂ of, e.g., 5 and 8 millimetres along the jaw member 2, 3 from its needle channel 11, thereby functioning as a measuring ruler to facilitate reproducing a desired suturing distance for a consistent, even and reliable suture.

In the following, reference is made to Figures 3A-C, in which the jaw members 2, 3 have been made invisible to illustrate the actuation of the locking elements 13. The locking elements 13 are biased to their locking position by respective locking element springs 131 that push a flange 132 of the elements 13 against locking element end stops 133 respectively connected to the jaw members 2, 3. This way, the locking element 13 is seated in its locking position in a stable manner. To move the respective locking elements 13 between their locking and release positions, the alternator system 80 comprises an alternator element 8. The alternator element 8 is tiltable about an axis 84 between a first position and a second position to alternately push the respective locking elements 13 out of their locking position and into their release position. The tilt axis 84 is stationary relative to the lower jaw member 3. In the first position, as shown in Figures 3A-B, the upper locking element 13 is in its locking position and the lower locking element 13 is in its release position. In the second position, as shown in Figure 3C, the upper locking element 13 is in its release position and the lower locking element 13 is in its locking position.

The alternator element 8 is moved between the first position and the second position by the trigger system 50, which comprises said button 5 and a trigger element 51 arranged to, upon pressing the button 5, engage the alternator element 8. If the alternator element 8 is in the first position, as shown in Figure 3B, the trigger element 51 is positioned to engage a first cam profile 81 of the alternator element 8. By engaging the first cam profile 81 with the trigger element 51, the alternator element 8 moves from the first position to the second position as illustrated stepwise in Figures 3B-C. As the button 5 returns to its neutral position, also the trigger element 51 returns to its neutral position but (contrary to the situation depicted in Figure 3B) the trigger element 51 is now positioned to, upon pressing the button 5 a second time, engage a second cam profile 82 of the alternator element 8 instead of the first cam profile 81. Then, upon actuating the trigger element 51 as indicated in Figure 3A by arrow B, the trigger element 51 engages the second cam profile 82 to rotate the alternator element 8 as indicated by arrow A₁, which in turn pushes the lower locking element 13 as indicated by arrow P₁. Similarly, when the trigger element 51 engages the first cam profile 81 as illustrated in Figure 3C, the alternator element 8 rotates as indicated by arrow A₂ and the upper locking element 13 is pushed as indicated by arrow P₂.

The alternator element 8 has a lower pushing portion 87 for directly engaging the lower locking element 13. To push the upper locking element 13, an intermediate rocker element 9 is provided. The rocker element 9 is elongate and, at one end, pivotably engages an upper pushing portion 88 of the alternator element 8. A locking element pusher axle 91, shown in Figure 2, keeps this end of the rocker element 9 in contact with the upper pushing portion 88 of the alternator element 8. At its other end, the rocker element 9 directly engages the upper locking element 13.

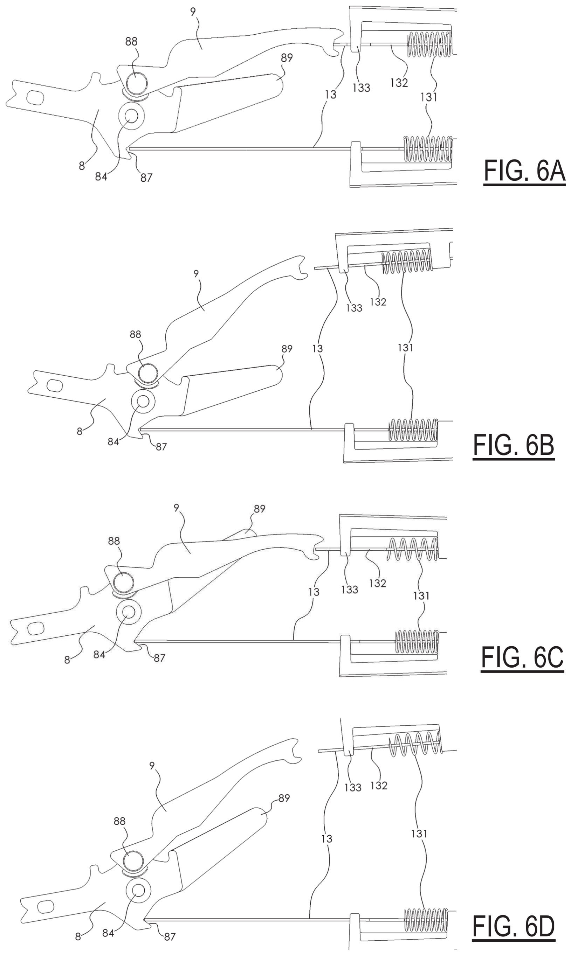

This rocker element 9 is provided to compensate for a difference between the distances between the alternator element 8 and the upper locking element 13 in respectively the closed state and the open state. This is illustrated by Figures 6A-D. In Figure 6A, the jaw members 2, 3 are in the closed state and the alternator element 8 is in its second position. The upper locking element spring 131 pushes the upper locking element 13 against the rocker element 9. In this situation, the flange 132 of the upper locking element 13 does not contact the upper locking element end stop 133. When moving the jaw members 2, 3 to the open state, as shown in Figure 6B, the upper jaw member 2 moves away from the alternator element 8, as the tilt axis 84 of the alternator element 8 is stationary relative to the lower jaw member 3. In the open state, the locking element pusher axle 91 and the upper pushing portion 88 of the alternator element 8 are spaced from a longitudinal axis of the upper jaw member 2. The rocker element 9 extends from the upper pushing portion 88 to the upper locking element 13 to bridge the distance between the upper pushing portion 88 and the upper locking element 13.

In Figure 6C, the jaw members 2, 3 are in the closed state and the alternator element 8 is in its first position. Again, when moving the jaw members 2, 3 to the open state, as shown in Figure 6D, the upper jaw member 2 moves away from the alternator element 8 and the rocker element 9 extends from the upper pushing portion 88 to the upper locking element 13. However, in the situation as shown in Figure 6D, the distance between the upper pushing portion 88 and the upper locking element 13 is larger than in the situation as shown in Figure 6B. Therefore, in Figure 6D, as the flange 132 of the upper locking element 13 rests against the upper locking element end stop 133, the rocker element 9 loses contact with the locking element 13.

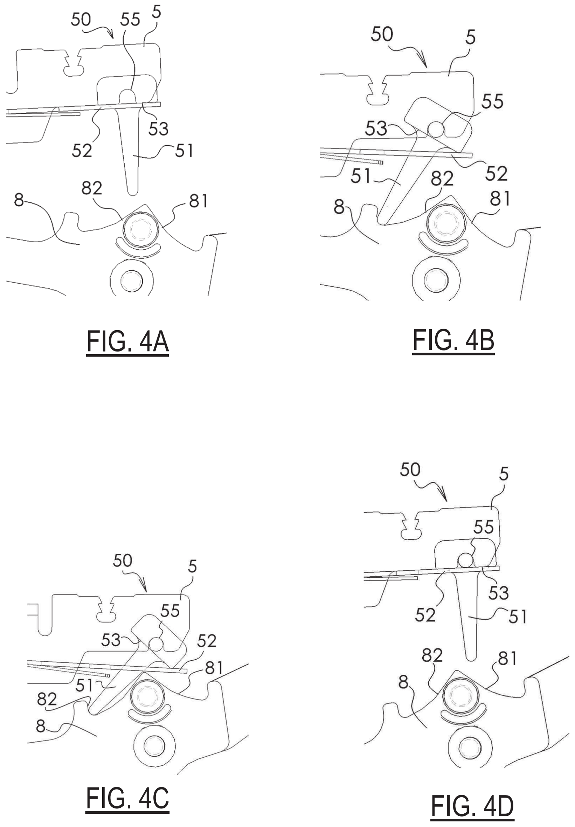

The movement of the trigger element 51 is illustrated in more detail in Figures 4A-D. In Figure 4A, the trigger element 51 is shown in its neutral position. The trigger element 51 is positioned to engage the second cam profile 82 on the alternator element 8. The trigger element 51 is biased to its neutral position by a leaf-spring 52, which presses against a flat surface 53 of the trigger element 51 to press the trigger element 51 against an engaging portion 55. Upon pressing the button 5, the trigger element 51 engages the second cam profile 82 and thereby rotates into a position in which the trigger element 51 is seated in the cam profile 82, as shown in Figure 4B. This rotation is possible as the spring pressure biasing the trigger element 51 to its neutral position is overcome by the triggering force. Due to the triggering force, the seated trigger element 51 pushes the alternator element 8 to its first tilt position, as shown in Figure 4C. As the button 5 returns to its neutral position, the trigger element 51 is lifted out of the second cam profile 82 and, due to the trigger-biasing leaf-spring 52, returns to its neutral position in which the trigger element 51 is now positioned to engage the first cam profile 81 of the alternator element 8, as shown in Figure 4D.

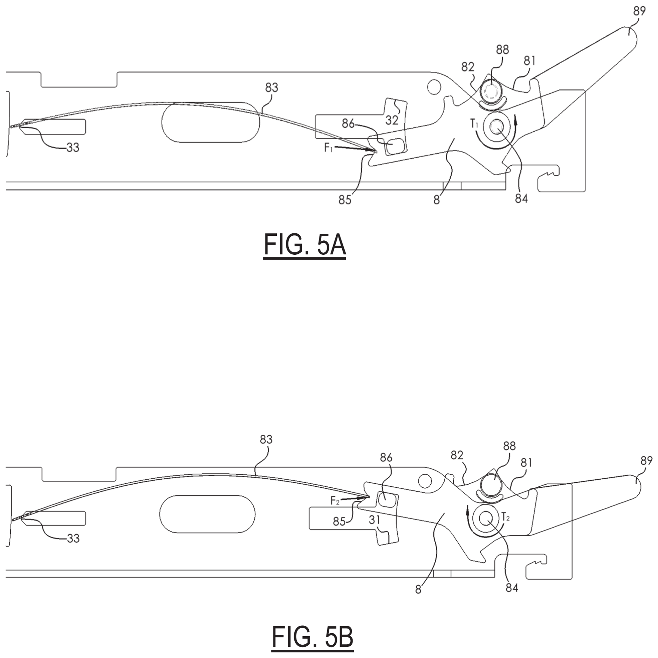

To bias the alternator element 8 to alternately the first position and the second position, a leaf-spring 83 is provided, as illustrated in more detail in Figures 5A-B, which show the alternator element 8 in its respective positions. The leaf-spring 83 is preloaded and arranged between an alternating seating portion 85 on the alternator element 8 at one end and, at its other end, a stationary seating portion 33 connected to the lower jaw member 3 to support this other end of the leaf-spring 83 stationary relative to the lower jaw member 3. This way, the leaf-spring 83 is arranged to push against the alternator element 8 with a force to apply a torque to the alternator element 8 about its axis 84. In the first position, as illustrated in Figure 5A, the force as indicated by arrow F₁ results in a torque in a first direction as indicated by arrow T₁. In the second position, as illustrated in Figure 5B, the force as indicated by arrow F₂ results in a torque in a second direction as indicated by arrow T₂. The direction of the torque depends on the position of the alternating seating portion 85 relative to a virtual straight line extending from the stationary seating portion 33 to the tilting axis 84 of the alternator element 8. If the alternating seating portion 85 is below this line, the alternator element 8 is biased to its first position, in which a cam portion 86 on the alternator element 8 is pushed by the leaf-spring 83 against a first alternator end stop 31 connected to the lower jaw member 3. If the alternating seating portion 85 is above the virtual line from the stationary seating portion 33 to the axis 84, the alternator element 8 is biased to its second position, in which the cam portion 86 on the alternator element 8 is pushed by the leaf-spring 83 against a second alternator end stop 32 connected to the lower jaw member 3. As such, bi-stability of the system 80 is ensured. If the alternating seating portion 85 would be motionless exactly on said virtual line, the system 80 would be in an unstable equilibrium. The configuration of the trigger system 50 ensures that, upon pressing the button 5, the alternator element 8 is moved through this unstable equilibrium point.

In the following, the loading of a needle end 102 into one of the needle holders 10 at the start of a suturing operation is described with reference to Figures 8A-C. As mentioned, the plate-like end of the elongate locking element 13 is provided with a needle-locking opening 15 for receiving and accommodating the needle end 102. More specifically, the plate-like end of the elongate locking element 13 is formed by a pair of prong parts 151 defining the wide section 16 and the narrow section 17 of the locking opening 15 therebetween. In the locking position of the locking element 13, the narrow section 17 of the locking opening 15 is aligned with the needle channel 11 as shown in Figure 7C. The prong parts 151 are outwardly bendable such that in the locking position, upon inserting a needle end 102 into the locking opening 15 via its narrow section 17 as indicated by arrow N in Figure 8B, the needle end 102 pushes the prong parts 151 away from each other as indicated by arrows L₁. Once the needle end 102 is inserted and the groove 103 at the needle end 102 is aligned with the plate-like end of the locking element 13, the prong parts 151 snap back to their locking state as indicated by arrows L₂ in Figure 8C. In the locking state, the prong parts 151 are received in the groove 103 and thereby lock the needle end 13. As such, a needle 100 can be loaded into the needle holder 10 even if the locking element 13 is in its locking position. This is convenient, as the needle 100 can be loaded without the need to operate the alternator system 80. For example, first the needle 100 may be positioned in either one of the holders 10 but not yet locked, after which the jaw members 2, 3 can be brought to the closed state, in which either end 102 of the needle 100 is received in a respective needle holder 10, and which automatically locks the needle end 102 in the holder 10 whose locking element 13 was already in the locking position.

After the suturing operation, the needle 100 is to be released from the surgical suturing device 1. Thereto, as shown throughout the drawings, the alternator element 8 comprises a toggle portion 89 for tilting the alternator element 8 also in the open state of the jaw members 2, 3, i.e., without pressing the button 5. When tilting the alternator element 8 in the open state, the needle 100 is released on one end without also being locked at its other end. This way, the needle 100 can be fully released from the suturing device 1. For safety, to prevent unintended tilting of the alternator element 8 via the toggle portion 89, the toggle portion 89 is positioned between the jaw members 2, 3 and extends, in the closed state, exclusively within an outer contour of the pair of jaw members 2, 3. As such, the toggle portion 89 is positioned such that it can only be operated in the open state of the jaw members 2, 3.

The drawings and the above description serve to illustrate specific embodiments of the invention and do not limit the scope of protection defined by the appended claims.

The present disclosure further includes the following embodiments:
1. A surgical suturing device for passing a double-ended surgical needle back and forth, the device comprising:
   - a pair of jaw members movable relative to each other between an open state and a closed state, wherein each of the jaw members is provided with a needle-locking element that is movable between a locking position and a release position for alternately locking and releasing a respective end of the needle;
   - an alternator system arranged to move the needle-locking elements between their locking and release positions for locking one end of the needle and releasing the other end of the needle, wherein the alternator system comprises an alternator element that is movable from a first position to a second position for moving one of the needle-locking elements to its release position, and from the second position to the first position for moving the other needle-locking element to its release position,
   characterised by an alternator spring arranged to bias the alternator element to alternately the first position and the second position.
2. Surgical suturing device according to embodiment 1, wherein the alternator spring is preloaded.
3. Surgical suturing device according to embodiment 1 or 2, wherein the alternator spring is a leaf-spring.
4. Surgical suturing device according to embodiment 1, 2 or 3, wherein the alternator spring is arranged to push against the alternator element.
5. Surgical suturing device according to any of the preceding embodiments, wherein the alternator element is tiltable about a tilt axis between the first position and the second position.
6. Surgical suturing device according to embodiment 5, wherein the alternator spring is arranged to apply a torque to the alternator element about the tilt axis in a first torque direction in the first position and, in the second position, in a second torque direction opposite to the first torque direction.
7. Surgical suturing device according to embodiment 5 or 6, wherein the tilt axis is perpendicular to a plane spanned by the pair of jaw members.
8. Surgical suturing device according to any of the preceding embodiments, further comprising at least one end stop surface, wherein the alternator element is arranged to rest against the least one end stop surface in the first position and/or the second position.
9. Surgical suturing device according to any of the preceding embodiments, further comprising a trigger system arranged to move the alternator element between the first position and the second position.
10. Surgical suturing device according to embodiment 9, wherein the alternator element comprises a first cam profile and a second cam profile, wherein the trigger system comprises a trigger element arranged to alternately engage the first cam profile to move the alternator element from the first position to the second position, and the second cam profile to move the alternator element from the second position to the first position.
11. Surgical suturing device according to embodiment 10, wherein the trigger system further comprises a trigger spring arranged to bias the trigger element to a neutral position in which the trigger element is positioned to engage the first cam profile in the first position of the alternator element and to engage the second cam profile in the second position of the alternator element.
12. Surgical suturing device according to embodiment 11, wherein the trigger element comprises at least one flat engaging portion, wherein the trigger spring is arranged to press the flat engaging portion of the trigger element against a further flat engaging portion to bias the trigger element to the neutral position.
13. Surgical suturing device according to embodiment 12, wherein the trigger spring is a leaf-spring comprising a trigger-engaging surface arranged to press against the flat engaging portion of the trigger element to bias the trigger element to the neutral position.
14. Surgical suturing device according to any of the preceding embodiments 9-13, wherein the trigger system comprises an operating element arranged on one of the jaw members at a side facing away from the other jaw member, wherein the operating element is manually movable between a neutral position and a triggered position for operating the trigger system to move the alternator element between the first position and the second position.
15. Surgical suturing device according to embodiment 14, comprising an opening spring arranged to bias the pair of jaw members to the open state with a first force, and an operating spring arranged to bias the operating element to the neutral position with a second force, wherein the first force is smaller than the second force.
16. Surgical suturing device according to embodiment 15, wherein the operating spring is pretensioned between the operating element and the one jaw member on which the operating element is arranged, wherein the opening spring is pretensioned between the operating element and said other jaw member.
17. Surgical suturing device according to any of the preceding embodiments 9-16, further comprising a toggle element arranged for manually moving the alternator element between the first position and the second position in the open state of the pair of jaw members.
18. Surgical suturing device according to embodiment 17, wherein the toggle element comprises a manually operable end, positioned between the jaw members for manually operating the toggle element in the open state.
19. Surgical suturing device according to embodiment 18, wherein the operable end extends exclusively within an outer contour of the pair of jaw member in the closed state.
20. Surgical suturing device according to any of the preceding embodiments, wherein the surgical suturing device is a hand-held surgical suturing device.
21. Surgical suturing device according to any of the preceding embodiments, wherein each of the needle-locking elements is biased to its locking position.
22. Surgical suturing device according to any of the preceding embodiments, wherein the alternator system is arranged to alternately push directly against either needle-locking element during movement of the alternator element between the first position and the second position to move the respective needle-locking element to its release position.
23. Surgical suturing device according to any of the preceding embodiments, wherein the alternator system further comprises a rocker element arranged between the alternator element and one of the needle-locking elements to push against the respective needle-locking element.
24. Surgical suturing device according to embodiment 23, wherein the alternator element engages the rocker element at an alternator engagement point which, in the open state, is spaced from a longitudinal axis of the jaw member that is provided with the respective needle-locking element.
25. Surgical suturing device according to embodiment 24, wherein the rocker element extends from the alternator engagement point to the longitudinal axis of the jaw member.

## Claims

1. A surgical suturing device (1) for passing a double-ended surgical needle (100) back and forth, the device (1) comprising:
- a pair of jaw members (2, 3) movable relative to each other between an open state and a closed state, wherein each of the jaw members is provided with a needle-locking element (13) that is movable between a locking position and a release position for alternately locking and releasing a respective end (102) of the needle (100);
- an alternator system (80) arranged to move the needle-locking elements (13) between their locking and release positions for locking one end (102) of the needle (100) and releasing the other end (102) of the needle (100), wherein the alternator system (80) comprises an alternator element (8) that is tiltable about a tilt axis (84) from a first position to a second position for moving one of the needle-locking elements (13) to its release position, and from the second position to the first position for moving the other needle-locking element (13) to its release position;
- a preloaded alternator spring (83) arranged to exert a force on the alternator element (8) in the first position and the second position to bias the alternator element (8) to alternately the first position and the second position.

2. Surgical suturing device according to claim 1, wherein the alternator spring (83) is a leaf-spring.

3. Surgical suturing device according to claim 1 or 2, wherein the alternator spring (83) is arranged to push against the alternator element (8).

4. Surgical suturing device according to claim 1, 2 or 3, wherein the alternator spring (83) is arranged to apply a torque to the alternator element (8) about the tilt axis (84) in a first torque direction (T₁) in the first position and, in the second position, in a second torque direction (T₂) opposite to the first torque direction (T₁).

5. Surgical suturing device according to claim 1, 2, 3 or 4, wherein the tilt axis (84) is perpendicular to a plane spanned by the pair of jaw members (2, 3).

6. Surgical suturing device according to any of the preceding claims, further comprising at least one end stop surface (31, 32), wherein the alternator element (8) is arranged to rest against the least one end stop surface (31, 32) in the first position and/or the second position.

7. Surgical suturing device according to any of the preceding claims, further comprising a trigger system (50) arranged to move the alternator element (8) between the first position and the second position.

8. Surgical suturing device according to claim 7, wherein the alternator element (8) comprises a first cam profile (81) and a second cam profile (82), wherein the trigger system (50) comprises a trigger element (51) arranged to alternately engage the first cam profile (81) to move the alternator element (8) from the first position to the second position, and the second cam profile (82) to move the alternator element (8) from the second position to the first position, wherein the trigger system (50) further comprises a trigger spring (52) arranged to bias the trigger element (51) to a neutral position in which the trigger element (51) is positioned to engage the first cam profile (81) in the first position of the alternator element (8) and to engage the second cam profile (82) in the second position of the alternator element (8).

9. Surgical suturing device according to claim 8, wherein the trigger element (51) comprises at least one flat engaging portion (53), wherein the trigger spring (52) is arranged to press the flat engaging portion (53) of the trigger element (51) against a further flat engaging portion to bias the trigger element (51) to the neutral position.

10. Surgical suturing device according to claim 9, wherein the trigger spring (52) is a leaf-spring comprising a trigger-engaging surface arranged to press against the flat engaging portion (53) of the trigger element (51) to bias the trigger element (51) to the neutral position.

11. Surgical suturing device according to any of the preceding claims 7-10, further comprising a toggle element (89) arranged for manually moving the alternator element (8) between the first position and the second position in the open state of the pair of jaw members (2, 3).

12. Surgical suturing device according to claim 11, wherein the toggle element (89) comprises a manually operable end, positioned between the jaw members (2, 3) for manually operating the toggle element (89) in the open state, wherein the operable end extends exclusively within an outer contour of the pair of jaw member (2, 3) in the closed state.

13. Surgical suturing device according to any of the preceding claims, wherein the alternator system (80) is arranged to alternately push directly against either needle-locking element (13) during movement of the alternator element (8) between the first position and the second position to move the respective needle-locking element (13) to its release position.

14. Surgical suturing device according to any of the preceding claims, wherein the alternator system (80) further comprises a rocker element (9) arranged between the alternator element (8) and one of the needle-locking elements (13) to push against the respective needle-locking element (13), wherein the alternator element (8) engages the rocker element (9) at an alternator engagement point (88) which, in the open state, is spaced from a longitudinal axis of the jaw member (2) that is provided with the respective needle-locking element (13).

15. Surgical suturing device according to claim 14, wherein the rocker element (9) extends from the alternator engagement point (88) to the longitudinal axis of the jaw member (2).
